# EUROPEAN PATENT APPLICATION

(11) **EP 4 692 800 A2**
(43) Date of publication of application: **11.02.2026**
(21) Application number: 25221799.7
(22) Date of filing: 10.05.2021
(51) Int. Cl.: G01N 33/569

(54) **METHODS AND KITS FOR DETECTING ADENO-ASSOCIATED VIRUSES**

(30) Priority: 12.05.2020 US 202063023629 P
(62) Divisional of application: 21733006.7
(71) Applicant: Lonza Houston, Inc., Pearland, Texas 77047 (US)
(72) Inventor: WANG, Peng, Pearland 77047 (US); GU, Bingnan, Pearland 77047 (US); SETH, Anandita, Pearland 77047 (US)
(74) Representative: Greiner, Elisabeth

(57) **Abstract**

The present disclosure provides methods and kits for detecting adeno-associated viruses. In some embodiments, the adeno-associated virus is Anc80. Detection methods include HPLC- and ELISA-based methods.

## Description

### FIELD OF THE INVENTION

The present disclosure provides methods and kits for detecting adeno-associated viruses. In some embodiments, the adeno-associated virus is Anc80. Detection methods include HPLC- and ELISA-based methods.

### BACKGROUND

Adeno-associated viruses (AAVs) have emerged as an important tool for gene therapy. In general, an AAV for gene therapy is an assembled viral particle, e.g., that lacks the native viral genes and only contains a sequence of interest, such as a gene of interest (GOI), resulting in a recombinant AAV (rAAV). The gene of interest is then delivered by the rAAV particle into the host cell. A typical process for producing a rAAV particle involves delivering at least two plasmids into a producer cell: a first plasmid containing the gene of interest flanked by inverted terminal repeat (ITR) sequences, which are found in the native AAV genome (the ITR/GOI plasmid); a second plasmid containing the AAV rep/cap genes to be expressed *in trans* for virus assembly (the rep/cap plasmid); and often a third plasmid containing helper genes from either adeno or herpes viruses (the helper plasmid). The producer cell then produces an assembled rAAV particle containing the ITR/GOI plasmid (i.e., a "packaged" rAAV), and the packaged rAAV can then be isolated and purified from the producer cell.

Purification of rAAVs typically involves: harvesting the producer cells; lysing the producer cells; subjecting the cell lysate to gradient purification (e.g., using a iodixanol gradient) and/or fast protein liquid chromatography (FPLC), which can be ion exchange, size exclusion, and/or affinity based; and buffer exchange and concentration, e.g., using tangential flow filtration. After purification, the rAAV titer is typically measured. Recombinant AAVs and methods of production and purification are further described, e.g., in Naso et al., BioDrugs 31(4):317-334 (2017) and WO 2018/150269.

Purification of AAVs, e.g., rAAVs from producer cells, can be a time-consuming and labor-intensive process. Since virus titration is typically not performed until after a substantially purified sample is obtained, e.g., after most or all of the purification steps, a low yield batch (e.g., low number of viral particles and/or low ratio of full/empty capsids) is not detected until a substantial amount of time and reagents have been spent, greatly increasing costs and reducing production efficiency.

A further challenge associated with AAV production is to develop optimized processes for different viral vectors on a large scale. The iterative optimization of both upstream and downstream processes utilize analytic tools to characterize and quantify viral products for in-process samples.

### SUMMARY OF THE INVENTION

In some embodiments, the disclosure provides a method for detecting adeno-associated virus serotype Anc80 in a sample, comprising: (a) contacting the sample with: (i) a capture reagent that binds an adeno-associated virus (AAVx) or an adeno-associated virus serotype 8 (AAV8); and (ii) a detection reagent that binds an adeno-associated virus (AAVx); (b) forming a binding complex comprising the capture reagent, the Anc80, and the detection reagent; and (c) detecting the binding complex, thereby detecting the Anc80 in the sample.

In additional embodiments, the disclosure provides a kit comprising: (a) a capture reagent that binds an adeno-associated virus (AAVx); and (b) a detection reagent that binds an adeno-associated virus (AAVx).

In further embodiments, the disclosure provides a method of determining capsid titer of an adeno-associated virus in a cell suspension or cell lysate, the method comprising: (a) subjecting the cell suspension or cell lysate to high-performance liquid chromatography (HPLC), wherein the HPLC is performed with a resin that binds to viral particle (VP) of the adeno-associated virus; and (b) detecting the VP in the cell suspension or cell lysate, thereby determining capsid titer of the adeno-associated virus.

### BRIEF DESCRIPTION OF THE DRAWINGS

The following drawings form part of the present specification and are included to further demonstrate exemplary embodiments of certain aspects of the present invention.
FIG. 1 illustrates an exemplary AAV purification process according to embodiments herein. The AAV producer cells are grown in a bioreactor and collected by depth filtration. The AAVs are isolated and purified by chromatography.
FIGS. 2A-2C show results of HPLC chromatography experiments described in Example 1. FIGS. 2A, 2B, and 2C show respectively the standard curves generated from samples of Anc80.CMV.eGFP, AAV2.CMV.eGFP, and AAV8.CMV.eGFP using the same HPLC column.
FIG. 3 shows the results of HPLC chromatography experiments described in Example 1. FIG. 3 shows HPLC chromatograms of samples obtained during or after various steps of the AAV purification process, i.e., cell suspension, cell lysate, clarification of lysate, tangential flow filtration (TFF) retentate, and final filtration.
FIG. 4 shows the results of AAV titer calculations described in Example 1. Full/empty capsid ratios were determined using capsid titer measured by HPLC (calculated from the results in FIG. 3) and genome titer measured by ddPCR.
FIG. 5 shows a standard curve of an ELISA performed according to Example 2, with samples of Anc80.CMV.eGFP.
FIG. 6 shows a 4-parameter logic regression calculation to determine the curve fit of the standard curve shown in FIG. 5.

### DETAILED DESCRIPTION OF THE INVENTION

The present disclosure relates to methods and kits for detecting an adeno-associated virus.

Unless otherwise defined herein, scientific and technical terms used in the present disclosure shall have the meanings that are commonly understood by one of ordinary skill in the art.

Unless otherwise required by context, singular terms shall include pluralities and plural terms shall include the singular. As used herein, "a" or "an" may mean one or more. As used herein, when used in conjunction with the word "comprising," the words "a" or "an" may mean one or more than one. As used herein, "another" or "a further" may mean at least a second or more.

Throughout this application, the term "about" is used to indicate that a value includes the inherent variation of error for the method/device being employed to determine the value, or the variation that exists among the study subjects. Typically, the term "about" is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20%, depending on the situation. In some embodiments, one of skill in the art will understand the level of variability indicated by the term "about," due to the context in which it is used herein. It should also be understood that use of the term "about" also includes the specifically recited value.

The use of the term "or" in the claims is used to mean "and/or," unless explicitly indicated to refer only to alternatives or the alternatives are mutually exclusive, although the disclosure supports a definition that refers to only alternatives and "and/or."

As used herein, the terms "comprising" (and any variant or form thereof, such as "comprise" and "comprises"), "having" (and any variant or form thereof, such as "have" and "has"), "including" (and any variant or form thereof, such as "includes" and "include") or "containing" (and any variant or form thereof, such as "contains" and "contain") are inclusive or open-ended and do not exclude additional, unrecited elements or method steps. It is contemplated that any embodiment discussed in this specification can be implemented with respect to any method and/or kit of the present disclosure.

The use of the term "for example" and its corresponding abbreviation "e.g." means that the specific terms recited are representative examples and embodiments of the disclosure that are not intended to be limited to the specific examples referenced or cited unless explicitly stated otherwise.

As used herein, "between" is a range inclusive of the ends of the range. For example, a number between x and y explicitly includes the numbers x and y, and any numbers that fall within x and y.

As referred to herein, "adeno-associated virus" or "AAV" refers to a small sized, replicative-defective nonenveloped virus or viral particle containing a single stranded DNA of the family *Parvoviridae* and the genus *Dependoparvovirus.* Adeno-associated virus also include ancestral AAVs (Anc AAVs). Non-limiting examples of AAV serotypes include Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127. In addition to these serotypes, AAV pseudotypes have been developed. An AAV pseudotype contains the capsid of a first serotype and the genome of a second serotype (e.g., the pseudotype AAV2/5 corresponds to an AAV with the genome of serotype AAV2 and the capsid of AAV5). Methods of producing derivatives, modifications, and/or pseudotypes of AAVs are described in, e.g., Asokan et al., Mol. Ther. 20(4):699-708 (2012).

As used herein, the "rep" gene refers to the art-recognized region of the AAV genome that encodes the replication proteins of the AAV, which are collectively required for replicating the viral genome. Rep also refers to functional homologues of AAV genes, such as the human herpesvirus 6 (HHV-6) rep gene, which is also known to mediate AAV DNA replication. In some embodiments, AAVs described herein encodes a rep coding region.

As used herein, the "cap" gene refers to the art-recognized region of the AAV genome that encodes the capsid proteins of the AAV. Illustrative and non-limiting examples of capsid proteins are the AAV capsid proteins VP1, VP2, and VP3. The AAV capsid proteins interact to form an AAV capsid. Cap genes used in the present disclosure can refer to the cap genes from any AAV serotype or a combination of serotypes.

As used in the context of AAVs, the term "recombinant" means that the AAV is the product of one or more procedures that results in an AAV that is distinct from a naturally-occurring AAV. A recombinant AAV, or rAAV, refers to a virus or viral particle comprising at least one AAV capsid protein and an encapsidated polynucleotide rAAV vector comprising a heterologous polynucleotide (i.e., a polynucleotide other than a naturally-occurring AAV genome, e.g., comprising a transgene, or gene of interest,to be delivered into a cell such as a mammalian cell). The rAAV particle can be any serotype, pseudotype, or derivative described herein (e.g., Anc80, AAV1, AAV-2, AAV3, AAV4, AAV5, AAV6, AAV7, AAV8, AAV9, AAV10, AAV11, AAV12, AAV13, AAV14, AAV15, AAV16, or derivatives, modifications, and/or pseudotypes thereof). The rAAV particle can also include a combination of serotypes.

As used herein, the term "titer" or "viral titer" refers to the concentration of a virus, e.g., an AAV provided herein. Viral titer can be expressed as the number of viral particles or the number of infectious units per mL. In some embodiments, viral titer is determined by measuring the concentration of a viral component, e.g., a viral capsid or genome.

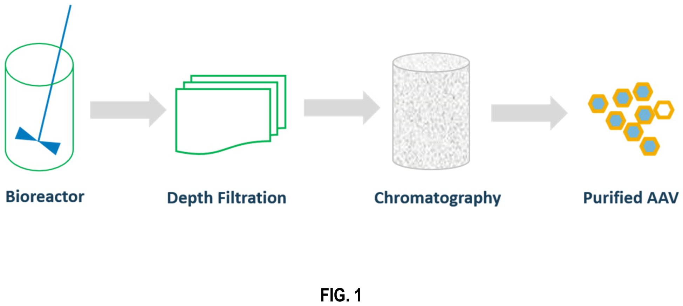

As described herein, purification of AAVs, e.g., rAAVs from producer cells, can be a time-consuming and labor-intensive process. An exemplary process for rAAV production and purification is illustrated in FIG. 1. In FIG. 1, producer cells are grown in a bioreactor to a desired cell density. The cells are collected by depth filtration and subjected to chromatography to obtain purified AAVs. Since virus titration is typically not performed until after a substantially purified sample is obtained, e.g., after most or all of the purification steps, a low yield batch (e.g., low number of viral particles and/or low ratio of full/empty capsids) is not detected until a substantial amount of time and reagents have been spent, greatly increasing costs and reducing production efficiency.

In some embodiments, the disclosure provides an analytical method to determine the AAV titer produced by a producer cell. In some embodiments, the method comprises measuring the AAV capsid titer. In some embodiments, the method comprises measuring the AAV genome titer. In some embodiments, the same method is used to detect different AAV serotypes, thereby providing an efficient and simplified procedure over current methods that require serotype-specific reagents, which may have different protocols. For example, for manufacturers of different serotypes of AAVs, the ability to utilize a single, streamlined process for detecting of any AAV serotype can reduce the number of equipment and maintenance thereof, allow equipment to be utilized more efficiently, lower reagent, processing, and labor costs, simplify personnel training, reduce the amount of different certifications, improve manufacturability and scaling, etc.

In some embodiments, the method is capable of detecting and/or titrating an AAV (e.g., Anc80) during the AAV purification process from a producer cell, as described herein. The method is advantageously capable of sensitive and specific detection and/or titration of AAV (e.g., Anc80), even in crude, unpurified samples, that may contain interfering components such as producer cell proteins and cellular debris, thereby providing a simple and efficient in-process AAV test. For example, the method can be performed in 90 min or less, 75 min or less, 60 min or less, or 45 in or less. In some embodiments, the method is capable of detecting and/or titrating an AAV (e.g., Anc80) in cell lysate. In some embodiments, the method is capable of detecting and/or titrating an AAV (e.g., Anc80) in a partially purified cell lysate. "Partially purified" cell lysate refers to a cell lysate that has been subjected to one or more downstream purification process steps after cell lysis, e.g., centrifugation, chromatography, buffer exchange, or filtration. In some embodiments, the method is capable of detecting and/or titrating an AAV (e.g., Anc80) in a purified sample, e.g., a sample that has been purified from an AAV producer cell as described herein. In some embodiments, the purified sample is free or substantially free of non-AAV particles.

### Chromatography Methods

In some embodiments, the disclosure provides an analytical method to determine the AAV titer produced by a producer cell. In some embodiments, the method is used to determine AAV titer at different points during the production process, e.g., at different time points during producer cell growth and/or during the AAV isolation and purification process described herein. In some embodiments, the method is capable of detecting and/or titrating AAV without lysing the producer cells. In some embodiments, the method comprises measuring the AAV capsid titer.

In some embodiments, the disclosure provides a method of determining capsid titer of an adeno-associated virus in a cell suspension or cell lysate the method comprising: (a) subjecting the cell suspension or cell lysate to high-performance liquid chromatography (HPLC), wherein the HPLC is performed with a resin that binds to a viral particle (VP) of the adeno-associated virus; and (b) detecting the VP in the cell suspension or cell lysate, thereby determining capsid titer of the adeno-associated virus.

In embodiments, the resins described herein are able to bind to capsid protein(s) of the viral particle.

In exemplary embodiments, the detection of the VP occurs spectrophotometrically, including for example by the use of absorbance measurements. Devices for measuring the absorbance of a solution following HPLC are well known and the art and readily configured to use as either part of an HPLC apparatus, or separately from an HPLC column. In embodiments, the detection of the VP of the adeno-associated virus occurs by obtaining the absorbance of the sample at about 270-290 nm, more suitably at about 280 nm, or specifically at 280 nm. This measurement can then be compared to calibration curve (either manually or as part of the instrument measurement) to provide an output of the amount of viral capsid in the sample.

In some embodiments, the cell suspension comprises producer cells suspended in growth media and/or buffer. In some embodiments, the cell lysate is an unpurified cell lysate, i.e., a cell lysate that has not been subjected to any downstream purification process after cell lysis. In other embodiments, the cell lysate is a partially purified cell lysate, e.g., a cell lysate that has been subjected to one or more downstream purification processes after cell lysis, as described herein. In some embodiments, the cell lysate comprises one or more impurities, which can include proteins, nucleic acids, polysaccharides, lipids, or other cellular components of the producer cell. In some embodiments, the impurity comprises a viral component that is not part of the assembled AAV particle. In some embodiments, the impurity comprises a partially assembled AAV particles. In some embodiments, the impurity comprises a misfolded viral protein or a malformed viral particle.

As described herein, in embodiments, the AAV comprises a fluorescent moiety. In some embodiments, the fluorescent moiety is a fluorescent dye. In some embodiments, the fluorescent moiety is incorporated into the AAV particle, e.g., incorporated into the capsid. In other embodiments, the fluorescent moiety is incorporated into a nucleic acid in the AAV. In further embodiments, the fluorescent moiety is incorporated into a protein or polypeptide in the AAV. In some embodiments, the fluorescent moiety is a fluorescent protein. In some embodiments, the AAV expresses the fluorescent protein. In some embodiments, the fluorescent protein is green fluorescent protein (GFP), blue fluorescent protein (BFP), red fluorescent protein (RFP), yellow fluorescent protein (YFP), cyan fluorescent protein (CFP), mCherry, mApple, mTurquoise, mVenus, mKO2, mKate2, or any variant or derivative thereof (e.g., eGFP). Additional fluorescent moieties and tags are known in the art, and include for example red fluorescent proteins such as TagRFP, mKate2, mRuby2, and Fusion Red, as well as other fluorescence proteins including for example monomerized (V206K) superfolder GFP, mTurquoise, Cerulean3, enhanced blue FP 2 (EBFP2), TagBFP, mNeonGreen, and monomerized Venus (A206K), etc. In embodiments where a fluorescence moiety or tag is utilized with the adeno-associate virus, the detection suitably comprises fluorescence detection. Devices for such fluorescence detection are known in the art and can readily be combined with HPLC devices and apparatuses. Fluorescent moieties are further described, e.g., in Jensen, The Anatomical Record 295(12):2031-2036 (2012). In some embodiments, the AAV is detected using fluorescence, e.g., via a fluorescence detector attached to the HPLC. Fluorescence detection, e.g., of VP, has higher sensitivity as compared with detection of absorbance at 260 nm and/or 280 nm. Fluorescence detection can also provide a simple and straightforward output compared with A260/A280 measurements, which require a ratio calculation. In some embodiments, the limit of detection of the methods described herein that utilize fluorescence detection or absorbance measurements is about 10⁶, about 10⁷, about 10⁸, about 10⁹, or about 10¹⁰ viral particles.

In some embodiments, the HPLC is performed with the resin contained in a column having a volume of about 0.1 mL to about 5.0 mL, about 0.1 mL to about 4.0 mL, about 0.1 mL to about 3.0 mL, about 0.1 mL to about 2.0 mL, about 0.1 mL to about 1.0 mL, about 0.1 mL to about 0.9 mL, about 0.2 mL to about 0.8 mL, about 0.3 mL to about 0.7 mL, or about 0.4 mL to about 0.6 mL. In some embodiments, the HPLC is performed with the resin contained in a column having a volume of about 0.1 mL, about 0.2 mL, about 0.3 mL, about 0.4 mL, about 0.5 mL, about 0.6 mL, about 0.7 mL, about 0.8 mL, about 0.9 mL, about 1.0 mL, about 1.5 mL, about 2.0 mL, about 2.5 mL, about 3.0 mL, about 3.5 mL, about 4.0 mL, about 4.5 mL, or about 5.0 mL.

In some embodiments, the resin is an affinity resin. Suitably, the resin comprises an antibody or variant thereof, including an antigen/epitope-binding portion thereof, an antibody fragment or derivative, an antibody analogue, an engineered antibody, or a substance that binds to an antigen in a similar manner to an antibody. In embodiments, the resin comprises at least one heavy or light chain complementarity determining region (CDR) of an antibody. In some embodiments, the resin comprises at least two CDRs from one or more antibodies. In exemplary embodiments, the resin comprises an antibody or antigen-binding fragment thereof.

In some embodiments, the resin binds an adeno-associated virus of any serotype or a broad array of AAV serotypes. As described herein, AAVx refers to such broad array of AAV serotypes. In some embodiments, the resin is capable of specifically binding any of Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127. In suitable embodiments, the AAV comprises Anc80. In some embodiments, the resin binds a capsid protein of the viral particle (VP) of the AAV. In embodiments, the resin binds an assembled AAV particle, and suitably, the resin binds a conformational epitope on the AAV capsid.

In some embodiments, the AAV titer (e.g., Anc80 titer) is determined by measuring the peak area on the HPLC chromatogram. In exemplary embodiments, the method further comprises measuring the AAV genome titer (e.g., Anc80 titer) by digital droplet PCR (ddPCR). Titration of viruses, e.g., AAVs, by ddPCR is described in Furuta-Hanawa et al., Hum. Gene Ther. Methods 30(4):127-136 (2019). In some embodiments, the method further comprises determining a ratio of full to empty capsids in the cell suspension or cell lysate based on the result of the AAV titers measured by HPLC and ddPCR.

As described herein, it has been surprisingly found that HPLC methods can be utilized to determine capsid titer or AAV serotypes, including Anc80, without the need for first purifying a cellular sample, and with the use of a small sample size (e.g., 10s-100s of mL of sample). This allows for a rapid and simple method of viral titer determination directly during processing, allowing for a fast determination regarding downstream processing.

The HPLC methods described herein can be utilized in combination with any bioreactor as well as any volume of bioreactor process. Exemplary reactor(s) include but are not limited to stirred tank, airlift, fiber, microfiber, hollow fiber, ceramic matrix, fluidized bed, fixed bed, and/or spouted bed bioreactors. As used herein, "reactor" can include a fermenter or fermentation unit, or any other reaction vessel and the term "reactor" is used interchangeably with "fermenter." The term fermenter or fermentation refers to both microbial and mammalian cultures. For example, in some aspects, an example bioreactor unit can perform one or more, or all, of the following: feeding of nutrients and/or carbon sources, injection of suitable gas (e.g., oxygen), inlet and outlet flow of fermentation or cell culture medium, separation of gas and liquid phases, maintenance of temperature, maintenance of oxygen and CO2 levels, maintenance of pH level, agitation (e.g., stirring), and/or cleaning/sterilizing. Example reactor units, such as a fermentation unit, may contain multiple reactors within the unit, for example the unit can have 1, 2, 3, 4, 5, 10, 15, 20, 25, 30, 35, 40, 45, 50, 60, 70, 80, 90, or 100, or more bioreactors in each unit and/or a facility may contain multiple units having a single or multiple reactors within the facility. In various embodiments, the bioreactor can be suitable for batch, semi fed-batch, fed-batch, perfusion, and/or a continuous fermentation processes. Any suitable reactor diameter can be used. In embodiments, the bioreactor can have a volume between about 100 mL and about 50,000 L. Non-limiting examples include a volume of 100 mL, 250 mL, 500 mL, 750 mL, 1 liter, 2 liters, 3 liters, 4 liters, 5 liters, 6 liters, 7 liters, 8 liters, 9 liters, 10 liters, 15 liters, 20 liters, 25 liters, 30 liters, 40 liters, 50 liters, 60 liters, 70 liters, 80 liters, 90 liters, 100 liters, 150 liters, 200 liters, 250 liters, 300 liters, 350 liters, 400 liters, 450 liters, 500 liters, 550 liters, 600 liters, 650 liters, 700 liters, 750 liters, 800 liters, 850 liters, 900 liters, 950 liters, 1000 liters, 1500 liters, 2000 liters, 2500 liters, 3000 liters, 3500 liters, 4000 liters, 4500 liters, 5000 liters, 6000 liters, 7000 liters, 8000 liters, 9000 liters, 10,000 liters, 15,000 liters, 20,000 liters, and/or 50,000 liters. Additionally, suitable reactors can be multi-use, single-use, disposable, or non-disposable and can be formed of any suitable material including metal alloys such as stainless steel (e.g., 316L or any other suitable stainless steel) and Inconel, plastics, and/or glass.

### Immunoassay Method

In some embodiments, the disclosure provides an immunoassay method for detecting and/or titrating an adeno-associated virus (AAV) in a sample. In some embodiments, the method is used to determine AAV titer at different points during the production process, e.g., at different time points during producer cell growth and/or during the AAV isolation and purification process described herein. In some embodiments, the method is capable of detecting and/or titrating AAV without lysing the producer cells. In some embodiments, the method comprises measuring the AAV capsid titer.

In some embodiments, the method is an immunoassay (e.g., an enzyme-linked immunosorbent assay or ELISA) for detecting and/or titrating an AAV (e.g., Anc80) in a sample. Immunoassays provide numerous advantages, such as high specificity and sensitivity, and can also be conducted in a high throughput format to assess multiple samples in one experiment. In some embodiments, the immunoassay is capable of detecting and/or titrating multiple AAV serotypes using the same capture and/or detection reagents. For example, the immunoassay can be conducted in a multi-well plate, and each well (or each group of wells) can correspond to a different AAV serotype.

In some embodiments, the sample is a cell suspension, i.e., comprising producer cells suspended in growth media and/or buffer. In some embodiments, the sample is an unpurified cell lysate, i.e., a cell lysate that has not been subjected to any purification process after cell lysis. In some embodiments, the sample is a partially purified cell lysate, e.g., a cell lysate that has been subjected to one or more purification processes after cell lysis, as described herein. In some embodiments, the sample comprises one or more impurities. In some embodiments, the impurity comprises a protein, nucleic acid, polysaccharide, lipid, or other cellular component of the producer cell. In embodiments, the impurity comprises a viral component that is not part of the assembled AAV particle. In other embodiments, the impurity comprises a partially assembled AAV particle. In some embodiments, the impurity comprises a misfolded viral protein or a malformed viral particle.

In exemplary embodiments, the disclosure provides a method for detecting an adeno-associated virus (AAV) in a sample, comprising: (a) contacting the sample with: (i) a capture reagent that binds an adeno-associated virus (AAVx); and (ii) a detection reagent that binds an adeno-associated virus (AAVx); (b) forming a binding complex comprising the capture reagent, the AAV, and the detection reagent; and (c) detecting the binding complex, thereby detecting the AAV in the sample.

In some embodiments, the AAV is any of the serotypes provided herein. In some embodiments, the AAV is Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, Anc127, or a combination thereof. In some embodiments, the AAV is Anc80.

In additional embodiments, the disclosure provides a method for detecting adeno-associated virus serotype Anc80 in a sample, comprising: (a) contacting the sample with: (i) a capture reagent that binds an adeno-associated virus (AAVx) or an adeno-associated virus serotype 8 (AAV8); and (ii) a detection reagent that binds an adeno-associated virus (AAVx) or an adeno-associated virus serotype 8 (AAV8); (b) forming a binding complex comprising the capture reagent, the Anc80, and the detection reagent; and (c) detecting the binding complex, thereby detecting the Anc80 in the sample.

Anc80 is a predicted ancestor of AAV serotypes 1, 2, 8, and 9 and was reconstructed by Zinn et al. as a potent gene therapy vector (see Zinn et al., Cell Reports 12:1056-1068 (2015) and Landegger et al., Nature Biotechnol. 35:280-284 (2017)). Anc80 clones include, e.g., Anc80L27, Anc80L65, Anc80L121. In some embodiments, the method provided herein is capable of detecting Anc80L27, Anc80L65, and Anc80L121.

### Capture and Detection Reagents

In some embodiments, the capture reagent is an antibody or variant thereof, including an antigen/epitope-binding portion thereof, an antibody fragment or derivative, an antibody analogue, an engineered antibody, or a substance that binds to an antigen in a similar manner to an antibody. In some embodiments, the capture reagent comprises at least one heavy or light chain complementarity determining region (CDR) of an antibody. In some embodiments, the capture reagent comprises at least two CDRs from one or more antibodies. In some embodiments, the capture reagent is an antibody or antigen-binding fragment thereof. In some embodiments, the capture reagent is a monoclonal antibody.

In exemplary embodiments, the capture reagent is immobilized on a surface. Suitable surfaces include, e.g., a particle (such as a bead), including a column prepared from such particles, as well as a plastic substrate such as a multi-well plate. In some embodiments, the surface comprises a multi-well plate, and the capture reagent is immobilized in a well of the multi-well plate. In some embodiments, the surface comprises a particle, and the capture reagent is immobilized on the particle. In some embodiments, the capture reagent comprises a conjugation moiety that is capable of reacting with its corresponding conjugation partner on the surface. Exemplary conjugation moiety-conjugation partner pairs include, but are not limited to, a receptor-ligand pair, complementary oligonucleotides, or cross-reactive moieties such as, e.g., thiol and maleimide or iodoacetamide; aldehyde and hydrazide; or azide and alkyne or cycloalkyne. In some embodiments, the capture reagent comprises biotin, and the surface comprises avidin or streptavidin.

In some embodiments, the capture reagent binds an adeno-associated virus of any serotype (AAVx). In some embodiments, the capture reagent is capable of specifically binding any of Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127. In some embodiments, the capture reagent binds Anc80. In embodiments, the capture reagent binds a capsid protein (VP) of the Anc80. In some embodiments, the capture reagent binds an assembled AAV particle. In some embodiments, the capture reagent binds a conformational epitope on the AAV capsid.

In additional embodiments, the capture reagent binds AAV8. In some embodiments, the capture reagent is capable of binding AAV8 and Anc80.

In some embodiments, the capture reagent binds to a capsid protein of a viral particle (VP) of AAV8 or a protein of Anc80. Suitably, the capture reagent binds the VP3 protein of AAV8 or the VP3 protein Anc80. In embodiments, the capture reagent binds to an epitope within residues 575-610, or within residues 580-600, or within residues 580-595 of the VP3 of AAV8 or the VP3 protein of Anc80. In suitable embodiments, the capture reagent binds to an epitope within residues 586-591 of the AAV8 VP3 protein. In other embodiments, the capture reagent binds to an epitope within residues 589-594 of the Anc80 VP3 protein. In some embodiments, the capture reagent binds to the sequence LQSANT (SEQ ID NO:1). In other embodiments, the capture reagent binds to the sequence LQQQNT (SEQ ID NO:2). In some embodiments, the capture reagent is AAV8 antibody clone ADK8.

Suitably, the detection reagent is an antibody or variant thereof, including an antigen/epitope-binding portion thereof, an antibody fragment or derivative, an antibody analogue, an engineered antibody, or a substance that binds to an antigen in a similar manner to an antibody. In some embodiments, the detection reagent comprises at least one heavy or light chain complementarity determining region (CDR) of an antibody. In some embodiments, the detection reagent comprises at least two CDRs from one or more antibodies. In exemplary embodiments, the detection reagent is an antibody or antigen-binding fragment thereof. In some embodiments, the detection reagent is a monoclonal antibody.

Suitably, the detection reagent binds an adeno-associated virus of any serotype (AAVx). In some embodiments, the detection reagent is capable of binding any of Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127. In some embodiments, the detection reagent binds Anc80. In some embodiments, the detection reagent binds a capsid protein of a VP of the Anc80. In some embodiments, the detection reagent binds an assembled AAV particle. In some embodiments, the detection reagent binds a conformational epitope on the AAV capsid.

In further embodiments, the detection reagent binds AAV8. In some embodiments, the detection reagent is capable of binding AAV8 and Anc80. In embodiments, the detection reagent binds to a capsid protein of a viral particle (VP) of AAV8 or a protein of Anc80, and suitably, the detection reagent binds the VP3 protein of AAV8 or the VP3 protein Anc80. In some embodiments, the detection reagent binds to an epitope within residues 575-610, or within residues 580-600, or within residues 580-595 of the VP3 of AAV8 or the VP3 protein of Anc80. In some embodiments, the detection reagent binds to an epitope within residues 586-591 of the AAV8 VP3 protein. In some embodiments, the detection reagent binds to an epitope within residues 589-594 of the Anc80 VP3 protein. In some embodiments, the detection reagent binds to the sequence LQSANT (SEQ ID NO:1). In some embodiments, the detection reagent binds to the sequence LQQQNT (SEQ ID NO:2). In some embodiments, the detection reagent is AAV8 antibody clone ADK8.

In additional embodiments, the capture reagent binds AAV8, and the detection reagent binds AAVx. In some embodiments, the capture reagent binds AAVx, and the detection reagent binds AAV8. In some embodiments, both the capture reagent and the detection reagent bind AAVx. In exemplary embodiments, both the capture reagent and the detection reagent are capable of binding any of Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127.

### Binding Complex Formation

In some embodiments, the binding complex comprising the capture reagent, the AAV (e.g., Anc80), and the detection reagent is formed in a single step. In other embodiments, the binding complex comprising the capture reagent, the AAV (e.g., Anc80), and the detection reagent is formed in one or more steps. In some embodiments, the binding complex is formed in solution, then immobilized to the surface. In some embodiments, the binding complex is formed by binding the AAV (e.g., Anc80) to the capture reagent immobilized on the surface, then binding the detection reagent to the AAV (e.g., Anc80) to form the binding complex on the surface. In some embodiments, the binding complex is formed by binding to the AAV (e.g., Anc80) to the capture reagent immobilized on the surface and to the detection reagent simultaneously. In other embodiments, the binding complex is formed by binding the AAV (e.g., Anc80) to the detection reagent in solution, then binding the AAV-detection reagent complex to the capture reagent on the surface. In additional embodiments, the binding complex is formed by binding the AAV (e.g., Anc80) to the capture reagent and the detection reagent in solution, then immobilizing the capture reagent to the surface as described herein.

### Detection

In embodiments, the binding complex is detected via a detectable moiety. For example, the detectable moiety is measured by spectrophotometry (color change), light scattering, optical absorbance, fluorescence, chemiluminescence, electrochemiluminescence, bioluminescence, phosphorescence, radioactivity, magnetic field, or combination thereof. In embodiments, the AAV titer (e.g., Anc80 titer) is determined by measuring the detectable moiety. In some embodiments, the detectable moiety is detectable in the presence of a substrate. Suitably, the detectable moiety is an enzyme that cleaves a substrate, and the detecting comprises detecting the cleaved substrate. In some embodiments, the detectable moiety comprises horseradish peroxidase (HRP), alkaline phosphatase (AP), glucose oxidase (GO), or beta galactosidase (BGAL or β-gal). Non-limiting examples of HRP substrates include, e.g., 3,3',5,5'-tetramethylbenzidine (TMB), 3,3'-diaminobenzidine (DAB), 2,2'-azino-bis(3-ethylbenzothiazoline-6-sulfonic acid) (ABTS), o-phenylenediamine dihydrochloride (OPD), AMPLEX^{®} Red; 3-amino-9-ethylcarbazole (AEC), homovanillic acid, luminol, and the SUPERSIGNAL^{™} ELISA, QUANTABLU^{™}, and QUANTARED^{™} substrates from ThermoFisher. Non-limiting examples of AP substrates include, e.g., p-nitrophenyl phosphate (PNPP) and the CDP-STAR^{™} and DYNALIGHT^{™} substrates from ThermoFisher. GO substrates include, e.g., glucose. BGAL substrates include, e.g., o-nitrophenyl-β-D-galactopyranoside (ONPG).

In some embodiments, the detection reagent comprises a detectable moiety, and detecting the binding complex comprises measuring the detectable moiety of the detection reagent. In embodiments, the detection reagent comprises a binding partner of a detectable moiety, and detecting the binding complex comprises contacting the detection reagent with the detectable moiety and measuring the detectable moiety bound to the detection reagent. In additional embodiments, the detection reagent and detectable moiety each comprises a binding partner in a binding pair. Suitable binding pairs are known in the art can include, but are not limited to, a receptor-ligand pair, complementary oligonucleotides, or cross-reactive moieties such as, e.g., thiol and maleimide or iodoacetamide; aldehyde and hydrazide; or azide and alkyne or cycloalkyne. In some embodiments, the detection reagent comprises biotin, and the detectable moiety comprises avidin or streptavidin. In some embodiments, the detection reagent comprises biotin, and the detectable moiety comprises horseradish peroxidase (HRP) conjugated to avidin or streptavidin. Suitably, detecting the binding complex comprises: binding the binding complex to HRP (e.g., via the biotin-avidin/streptavidin interaction on the detection reagent and HRP), contacting the HRP with a chromogenic or chemiluminescent HRP substrate, and detecting a change in color or a chemiluminescence signal. In some embodiments, the AAV titer (e.g., Anc80 titer) is determined by measuring the change in color and/or chemiluminescence signal.

### Kits

In some embodiments, the disclosure provides a kit for conducting the methods described herein, e.g., an immunoassay kit. In some embodiments, the disclosure provides a kit for detecting and/or titrating an adeno-associated virus (AAV), comprising: (a) a capture reagent that binds an adeno-associated virus (AAVx); and (b) a detection reagent that binds an adeno-associated virus (AAVx). The kits described herein also suitably include instructions for carrying out the various methods, including the ELISA-based methods, described herein.

In some embodiments, the kit is used to detect and/or titrate any AAV serotype described herein, e.g., Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, or Anc127. In some embodiments, the AAV is Anc80. Anc80 is further described herein.

In some embodiments, the disclosure provides a kit comprising: (a) a capture reagent that binds an adeno-associated virus (AAVx) or adeno-associated virus serotype 8 (AAV8); and (b) a detection reagent that binds an adeno-associated virus (AAVx) or adeno-associated virus serotype 8 (AAV8).

Capture reagents and detection reagents are further described herein. In some embodiments, the capture reagent is an antibody or antigen-binding fragment thereof. In some embodiments, the detection reagent is an antibody or antigen-binding fragment thereof.

In some embodiments, the kit comprises a capture surface. Capture surfaces can be any of the surfaces described herein. In some embodiments, the capture surface comprises a particle. In some embodiments, the capture surface comprises a plastic substrate such as a multi-well plate. In some embodiments, the capture reagent is immobilized on the capture surface. In some embodiments, the capture surface is a multi-well plate, and the capture reagent is immobilized on a well of the multi-well plate.

In some embodiments, the capture reagent and the capture surface are provided separately, and the kit further comprises a reagent for immobilizing the capture reagent to the capture surface. In some embodiments, the capture reagent comprises a conjugation moiety, and the capture surface comprises a conjugation partner thereof. Exemplary conjugation moiety-conjugation partner pairs include, but are not limited to, a receptor-ligand pair, complementary oligonucleotides, or cross-reactive moieties such as, e.g., thiol and maleimide or iodoacetamide; aldehyde and hydrazide; or azide and alkyne or cycloalkyne. In some embodiments, the capture reagent comprises biotin, and the capture surface comprises avidin or streptavidin attached thereto.

In some embodiments, the capture reagent binds an adeno-associated virus of any serotype (AAVx). In some embodiments, the capture reagent is capable of specifically binding any of Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127. In some embodiments, the capture reagent binds Anc80. In some embodiments, the capture reagent binds a capsid protein (VP) of the Anc80. In some embodiments, the capture reagent binds an assembled AAV particle. In some embodiments, the capture reagent binds a conformational epitope on the AAV capsid.

In some embodiments, the capture reagent binds AAV8. In some embodiments, the capture reagent is capable of binding AAV8 and Anc80. In some embodiments, the capture reagent binds to a capsid protein of a viral particle (VP) of AAV8 or a capsid protein of Anc80. In some embodiments, the capture reagent binds the VP3 protein of AAV8 or the VP3 protein Anc80. In some embodiments, the capture reagent binds to an epitope within residues 575-610, or within residues 580-600, or within residues 580-595 of the VP3 of AAV8 or the VP3 protein of Anc80. In some embodiments, the capture reagent binds to an epitope within residues 586-591 of the AAV8 VP3 protein. In some embodiments, the capture reagent binds to an epitope within residues 589-594 of the Anc80 VP3 protein. In some embodiments, the capture reagent binds to the sequence LQSANT (SEQ ID NO:1). In some embodiments, the capture reagent binds to the sequence LQQQNT (SEQ ID NO:2). In some embodiments, the capture reagent is AAV8 antibody clone ADK8.

In some embodiments, the detection reagent binds an adeno-associated virus of any serotype (AAVx). In some embodiments, the detection reagent is capable of binding any of Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127. In some embodiments, the detection reagent binds Anc80. In some embodiments, the detection reagent binds a capsid protein (VP) of the Anc80. In some embodiments, the detection reagent binds an assembled AAV particle. In some embodiments, the detection reagent binds a conformational epitope on the AAV capsid.

In some embodiments, the detection reagent binds AAV8. In some embodiments, the detection reagent is capable of binding AAV8 and Anc80. In some embodiments, the detection reagent binds to a capsid protein of a viral particle (VP) of AAV8 or a capsid protein of Anc80. In some embodiments, the detection reagent binds the VP3 protein of AAV8 or the VP3 protein Anc80. In some embodiments, the detection reagent binds to an epitope within residues 575-610, or within residues 580-600, or within residues 580-595 of the VP3 of AAV8 or the VP3 protein of Anc80. In some embodiments, the detection reagent binds to an epitope within residues 586-591 of the AAV8 VP3 protein. In some embodiments, the detection reagent binds to an epitope within residues 589-594 of the Anc80 VP3 protein. In some embodiments, the detection reagent binds to the sequence LQSANT (SEQ ID NO:1). In some embodiments, the detection reagent binds to the sequence LQQQNT (SEQ ID NO:2). In some embodiments, the detection reagent is AAV8 antibody clone ADK8.

In some embodiments, the capture reagent binds AAV8, and the detection reagent binds AAVx. In some embodiments, the capture reagent binds AAVx, and the detection reagent binds AAV8. In some embodiments, both the capture reagent and the detection reagent bind AAVx. In some embodiments, both the capture reagent and the detection reagent are capable of binding any of Anc80, Anc80L27, Anc80L65, Anc80L121, AAV1, AAV-2, AAV3, AAV4, AAV5, AAV6, AAV7, AAV8, AAV9, AAV10, AAV11, AAV12, AAV13, AAV14, AAV15, AAV16, AAV1.1, AAV2.5, AAV2i8, AAV2G9, AAV2tYF, AAV2-TT, AAV2-TT-S312N, AAV3B, AAV3B-S312N, AAV-LK3, AAV6.1, AAV6.3.1, AAV.7m8, AAV9.45, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, AAV.HSC16, AAV.rh8, AAV.rh10, AAV.rh20, AAV.rh32, AAV.rh33, AAV.rh39, AAV.rh74, AAV.RHM4-1, AAV.RHM15-1, AAV.RHM15-2, AAV.RHM15-3, AAV.RHM15-4, AAV.RHM15-5, AAV.RHM15-6, AAV.cy10, AAV.dj, AAV.po4, AAV.po6, AAV.hu26, AAV.hu37, AAV.PHP.B, Anc126, and Anc127.

In some embodiments, the detection reagent comprises a detectable moiety, e.g., that capable of being measured by spectrophotometry (color change), light scattering, optical absorbance, fluorescence, chemiluminescence, electrochemiluminescence, bioluminescence, phosphorescence, radioactivity, magnetic field, or combination thereof. In some embodiments, the detection reagent comprises a binding partner of a detectable moiety. In some embodiments, the detection reagent and detectable moiety each comprises a binding partner in a binding pair such as, e.g., a receptor-ligand pair, complementary oligonucleotides, or cross-reactive moieties such as, e.g., thiol and maleimide or iodoacetamide; aldehyde and hydrazide; or azide and alkyne or cycloalkyne. In some embodiments, the detection reagent comprises biotin, and the detectable moiety comprises avidin or streptavidin. In some embodiments, the detectable moiety comprises horseradish peroxidase (HRP), alkaline phosphatase (AP), glucose oxidase (GO), or beta galactosidase (BGAL or β-gal). In some embodiments, the detection reagent comprises biotin, and the detectable moiety comprises HRP conjugated to avidin or streptavidin.

In some embodiments, the capture reagent is lyophilized. In some embodiments, the capture reagent is provided in solution. In some embodiments, the detection reagent is lyophilized. In some embodiments, the detection reagent is provided in solution. In embodiments where the capture and/or detection reagent is lyophilized, the kit further suitably comprises a buffer for reconstituting or resuspending the lyophilized reagent. In some embodiments, the capture and/or detection reagents are provided separately from other components of the kit, e.g., according to their optimal shipping and/or storage temperatures.

In some embodiments, the kit further comprises one or more of a buffer, an assay stop solution, a calibration reagent, a detectable moiety capable of binding to the detection reagent, and a detectable substrate.

In some embodiments, the kit comprises one or more of an assay buffer, blocking buffer, coating buffer, wash buffer, reconstitution or resuspending buffer, and storage buffer. In some embodiments, the assay buffer comprises phosphate buffered saline, sodium carbonate, sodium bicarbonate, Tris, NaCl, TWEEN, or a combination thereof. In some embodiments, the kit comprises an assay stop solution. In some embodiments, the assay stop solution comprises sulfuric acid.

In some embodiments, the kit comprises a calibration reagent. In some embodiments, the calibration reagent comprises a known quantity of the AAV (e.g., Anc80). In some embodiments, the kit comprises multiple calibration reagents comprising a range of concentrations of the AAV (e.g., Anc80). In some embodiments, the multiple calibration reagents comprise concentrations of the AAV (e.g., Anc80) near the upper and lower limits of quantitation for a method performed using the kit. In some embodiments, the multiple calibration concentrations of the calibration reagents span the entire dynamic range of the method. In some embodiments, the multiple calibration reagents comprise multiple AAV serotypes, e.g., to calibrate the method for detecting different AAV serotypes. In some embodiments, the calibration reagent is a positive control reagent. In some embodiments, the calibration reagent is a negative control reagent. In some embodiments, the calibration reagent is lyophilized. In some embodiments, the calibration reagent is provided in solution.

In some embodiments, the kit comprises a detectable moiety capable of binding to the detection reagent. Detectable moieties are further described herein. In some embodiments, the detectable moiety comprises horseradish peroxidase (HRP), alkaline phosphatase (AP), glucose oxidase (GO), or beta galactosidase (BGAL or β-gal). In some embodiments, the kit further comprises a substrate for the detectable moiety. Exemplary substrates for the detectable moieties are provided herein. In some embodiments, the substrate is 3,3',5,5'-tetramethylbenzidine (TMB), 3,3'-diaminobenzidine (DAB), 2,2'-azino-bis(3-ethylbenzothiazoline-6-sulfonic acid) (ABTS), o-phenylenediamine dihydrochloride (OPD), AMPLEX^{®} Red; 3-amino-9-ethylcarbazole (AEC), homovanillic acid, luminol, and the SUPERSIGNAL^{™} ELISA, QUANTABLU^{™}, and QUANTARED^{™} substrates from ThermoFisher. In some embodiments, the substrate is p-nitrophenyl phosphate (PNPP) and the CDP-STAR^{™} and DYNALIGHT^{™} substrates from ThermoFisher. In some embodiments, the substrate is glucose. In some embodiments, the substrate is o-nitrophenyl-β-D-galactopyranoside (ONPG).

In some embodiments, the kit further comprises one or more of an assay consumable, e.g., assay modules, vials, tubes, liquid handling and transfer devices such as pipette tips, covers and seals, racks, and labels. In some embodiments, the kit further comprises instructions for performing an immunoassay to detect and/or titrate an AAV (e.g., Anc80). In some embodiments, the immunoassay comprises a method described herein.

### Exemplary Embodiments

Embodiment 1 is a method for detecting adeno-associated virus serotype Anc80 in a sample, comprising: contacting the sample with: (i) a capture reagent that binds an adeno-associated virus (AAVx) or an adeno-associated virus serotype 8 (AAV8); and (ii) a detection reagent that binds an adeno-associated virus (AAVx); forming a binding complex comprising the capture reagent, the Anc80, and the detection reagent; and detecting the binding complex, thereby detecting the Anc80 in the sample.

Embodiment 2 includes the method of embodiment 1, wherein the capture reagent and/or the detection reagent is an antibody or antigen-binding fragment thereof.

Embodiment 3includes the method of embodiment 1 or 2, wherein the capture reagent is immobilized on a surface.

Embodiment 4 includes the method of embodiment 3, wherein the capture reagent comprises biotin, and the surface comprises avidin or streptavidin.

Embodiment 5 includes the method of embodiment 3 or 4, wherein the surface is a multi-well plate, and the capture reagent is immobilized in a well of the multi-well plate.

Embodiment 6 includes the method of any of embodiments 1 to 5, wherein the capture reagent binds AAVx.

Embodiment 7 includes the method of any of embodiments 1 to 5, wherein the capture reagent binds AAV8.

Embodiment 8 includes the method of any of embodiments 1 to 7, wherein the capture reagent binds to a viral particle (VP) of the Anc80.

Embodiment 9 includes the method of embodiment 7, wherein the capture reagent binds to VP3.

Embodiment 10 includes the method of embodiment 9, wherein the capture reagent binds to an epitope within residues 580 to 600 of VP3.

Embodiment 11 includes the method of embodiment 10, wherein the capture reagent binds to LQSANT (SEQ ID NO:1).

Embodiment 12 includes the method of any of embodiments 1 to 11, wherein the detection reagent binds AAVx.

Embodiment 13 includes the method of any of embodiments 1 to 11, wherein the detection reagent binds AAV8.

Embodiment 14 includes the method of any of embodiments 1 to 13, wherein the detection reagent binds to a viral protein (VP) of the Anc80.

Embodiment 15 includes the method of any of embodiments 1 to 14, wherein the detection reagent comprises a binding partner of a detectable moiety.

Embodiment 16 includes the method of embodiment 15, wherein the detection reagent comprises biotin, and the detectable moiety comprises horseradish peroxidase conjugated to avidin or streptavidin.

Embodiment 17 includes the method of embodiment 1, wherein the capture reagent binds AAV8, and the detection reagent binds AAVx.

Embodiment 18 is a kit comprising: a capture reagent that binds an adeno-associated virus (AAVx) or adeno-associated virus serotype 8 (AAV8); and a detection reagent that binds an adeno-associated virus (AAVx).

Embodiment 19 includes the kit of embodiment 18, wherein the capture reagent and/or the detection reagent is an antibody or antigen-binding fragment thereof.

Embodiment 20 includes the kit of embodiment 18 or 19, further comprising a capture surface.

Embodiment 21 includes the kit of embodiment 20, wherein the capture reagent comprises biotin, and the capture surface comprises avidin or streptavidin attached thereto.

Embodiment 22 includes the kit of embodiment 20 or 21, wherein the capture surface is a multi-well plate, and the capture reagent is immobilized on a well of the multi-well plate.

Embodiment 23 includes the kit of any of embodiments 18 to 22, wherein the capture reagent binds AAVx.

Embodiment 24 includes the kit of any of embodiments 18 to 22, wherein the capture reagent binds AAV8.

Embodiment 25 includes the kit of any of embodiments 18 to 24, wherein the capture reagent binds to a viral protein (VP) of the Anc80.

Embodiment 26 includes the kit of embodiment 24, wherein the capture reagent binds to VP3.

Embodiment 27 includes the kit of embodiment 26, wherein the capture reagent binds to an epitope within residues 580 to 600 of VP3.

Embodiment 28 includes the kit of embodiment 27, wherein the capture reagent binds to LQSANT (SEQ ID NO:1).

Embodiment 29 includes the kit of any of embodiments 18 to 28, wherein the detection reagent binds AAVx.

Embodiment 30 includes the kit of any of embodiments 18 to 28, wherein the detection reagent binds AAV8.

Embodiment 31 includes the kit of any of embodiments 18 to 30, wherein the detection reagent binds to a capsid protein (VP) of the Anc80.

Embodiment 32 includes the kit of any of embodiments 18 to 31, further comprising a detectable moiety, and the detection reagent comprises a binding partner of the detectable moiety.

Embodiment 33 includes the kit of embodiment 32, wherein the detection reagent comprises biotin, and the detectable moiety comprises horseradish peroxidase conjugated to avidin or streptavidin.

Embodiment 34 includes the kit of any of embodiments 18 to 33, wherein the capture reagent, the detection reagent, or both, are lyophilized.

Embodiment 35 includes the kit of any of embodiments 18 to 33, wherein the capture reagent, the detection reagent, or both, are provided in solution.

Embodiment 36 includes the kit of any of embodiments 18 to 35, further comprising one or more of a buffer, an assay stop solution, a calibration reagent, a detectable moiety capable of binding to the detection reagent, and a detectable substrate.

Embodiment 37 includes the kit of embodiment 18, wherein the capture reagent binds AAV8, and the detection reagent binds AAVx.

Embodiment 38, is a method of determining capsid titer of an adeno-associated virus in a cell suspension or cell lysate, the method comprising: subjecting the cell suspension or cell lysate to high-performance liquid chromatography (HPLC), wherein the HPLC is performed with a resin that binds to a viral protein (VP) of the adeno-associated virus; and detecting the VP in the cell suspension or cell lysate, thereby determining capsid titer of the adeno-associated virus.

Embodiment 39 includes the method of embodiment 38, wherein the detecting comprises spectrophotometrically detecting capsid protein at 280 nm.

Embodiment 40 includes the method of embodiment 38, wherein the adeno-associated virus comprises a fluorescent moiety, and the detecting comprises fluorescence detection.

Embodiment 41 includes the method of any of embodiments 38-40, wherein the cell lysate is an unpurified cell lysate or a partially purified cell lysate.

Embodiment 42 includes the method of any of embodiments 38-41, wherein the HPLC is performed with the resin contained in a column having a volume of about 0.1 to about 1.0 mL.

Embodiment 43 includes the method of any of embodiments 38-42, wherein the resin comprises an antibody or antigen-binding fragment thereof.

Embodiment 44 includes the method of any of embodiments 38-43, wherein the adeno-associated virus comprises Anc80.

### EXAMPLES

### Example 1. HPLC Assay

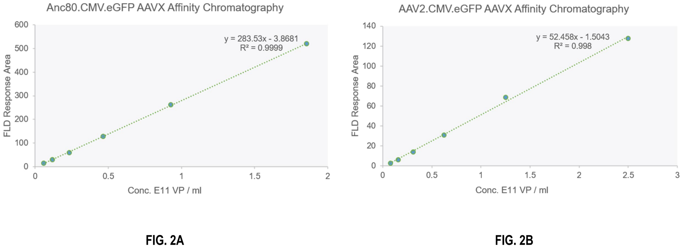

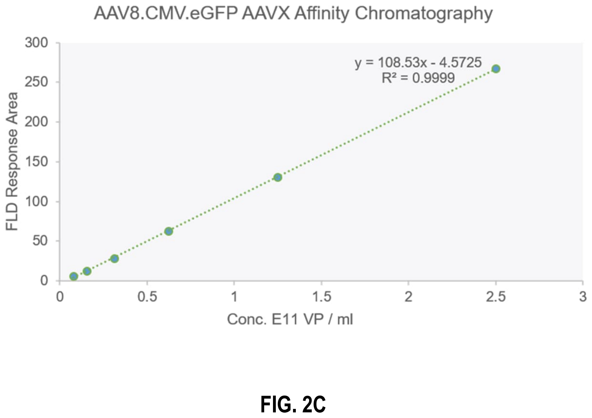

A broad-spectrum AAV (AAVX) affinity column was prepared by packing POROS^{™} CAPTURESELECT^{™} AAVX Affinity Resin (ThermoFisher) into a 50 × 4.6 mm HPLC column by Princeton Chromatography, Inc. An Agilent 1260 Infinity II Bio-Inert LC System with Fluorescence Detector was used to evaluate the AAVX affinity column with various AAV serotype samples, including Anc80.CMV.eGFP, AAV2.CMV.eGFP, and AAV8.CMV.eGFP. As shown in FIGS. 2A-2C, which depict standard curves generated respectively for Anc80.CMV.eGFP, AAV2.CMV.eGFP, and AAV8.CMV.eGFP, response linearity R² > 0.99 was observed for all three AAV serotypes, with a lower limit of detection of approximately 6 × 10⁸ total viral particles.

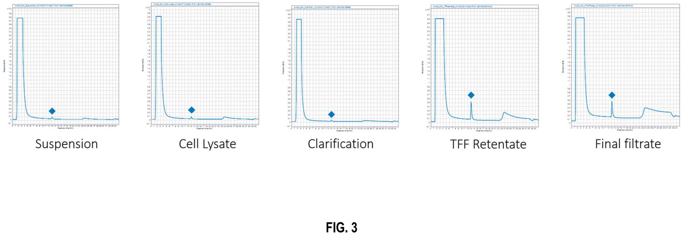

The HPLC method was further tested with in-process samples of Anc80. A 50 L bioreactor producing Anc80.CMV.eGFP was tested using the HPLC method during various steps of the purification process. As shown in FIG. 3, the Anc80.CMV.eGFP capsid peak was successfully detected in all tested steps: cell suspension, cell lysate, after lysate clarification, after tangential flow filtration (TFF), and after the final filtration step.

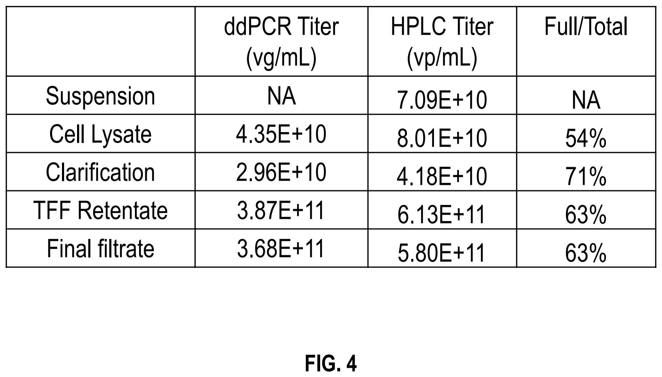

Titers of the in-process Anc80 samples were determined based on linear regression analysis. The titers were also determined using ddPCR, and the full/empty capsid ratios of the in-process samples were calculated as shown in FIG. 4.

### Example 2. Immunoassay

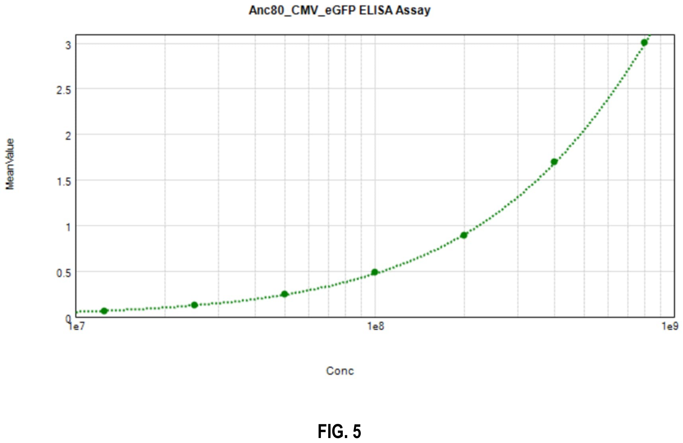

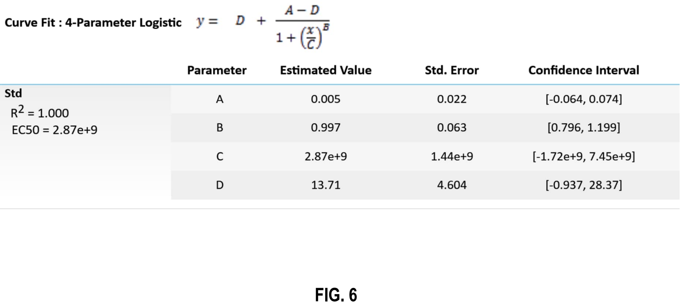

An ELISA assay for Anc80 was developed using an anti-AAV8 capture antibody and the CAPTURESELECT^{™} Biotin Anti-AAVX Conjugate (ThermoFisher) as detection antibody. A standard curve for Anc80.CMV.eGFP was generated using a 4-parameter logistic regression with R² > 0.99 (see FIG. 5, standard curve, and FIG. 6, regression calculation).

The ELISA assay was also applied to in-process samples from a 50 L bioreactor producing Anc80.CMV.eGFP. The titers of in-process samples are measured, and the results are comparable with titers measured by other methods.

All references cited herein, including patents, patent applications, papers, textbooks and the like, and the references cited therein, to the extent that they are not already, are hereby incorporated herein by reference in their entirety.

In view of the foregoing, it will be appreciated that the invention described herein inter alia relates to the following items:
1. A method for detecting adeno-associated virus serotype Anc80 in a sample, comprising:
   (a) contacting the sample with: (i) a capture reagent that binds an adeno-associated virus (AAVx) or an adeno-associated virus serotype 8 (AAV8); and (ii) a detection reagent that binds an adeno-associated virus (AAVx);
   (b) forming a binding complex comprising the capture reagent, the Anc80, and the detection reagent; and
   (c) detecting the binding complex, thereby detecting the Anc80 in the sample.
2. The method of item 1, wherein the capture reagent and/or the detection reagent is an antibody or antigen-binding fragment thereof.
3. The method of item 1 or 2, wherein the capture reagent is immobilized on a surface.
4. The method of item 3, wherein the capture reagent comprises biotin, and the surface comprises avidin or streptavidin.
5. The method of item 3 or 4, wherein the surface is a multi-well plate, and the capture reagent is immobilized in a well of the multi-well plate.
6. The method of any one of items 1 to 5, wherein the capture reagent binds AAVx.
7. The method of any one of items 1 to 5, wherein the capture reagent binds AAV8.
8. The method of any one of items 1 to 7, wherein the capture reagent binds to a viral particle of the Anc80.
9. The method of item 7, wherein the capture reagent binds to VP3.
10. The method of item 9, wherein the capture reagent binds to an epitope within residues 580 to 600 of VP3.
11. The method of item 10, wherein the capture reagent binds to LQSANT (SEQ ID NO:1).
12. The method of any one of items 1 to 11, wherein the detection reagent binds AAVx.
13. The method of any one of items 1 to 11, wherein the detection reagent binds AAV8.
14. The method of any one of items 1 to 13, wherein the detection reagent binds to a viral particle of the Anc80.
15. The method of any one of items 1 to 14, wherein the detection reagent comprises a binding partner of a detectable moiety.
16. The method of item 15, wherein the detection reagent comprises biotin, and the detectable moiety comprises horseradish peroxidase conjugated to avidin or streptavidin.
17. The method of item 1, wherein the capture reagent binds AAV8, and the detection reagent binds AAVx.
18. A kit comprising:
   (a) a capture reagent that binds an adeno-associated virus (AAVx) or adeno-associated virus serotype 8 (AAV8); and
   (b) a detection reagent that binds an adeno-associated virus (AAVx).
19. The kit of item 18, wherein the capture reagent and/or the detection reagent is an antibody or antigen-binding fragment thereof.
20. The kit of item 18 or 19, further comprising a capture surface.
21. The kit of item 20, wherein the capture reagent comprises biotin, and the capture surface comprises avidin or streptavidin attached thereto.
22. The kit of item 20 or 21, wherein the capture surface is a multi-well plate, and the capture reagent is immobilized on a well of the multi-well plate.
23. The kit of any one of items 18 to 22, wherein the capture reagent binds AAVx.
24. The kit of any one of items 18 to 22, wherein the capture reagent binds AAV8.
25. The kit of any one of items 18 to 24, wherein the capture reagent binds to a capsid protein (VP) of the Anc80.
26. The kit of item 24, wherein the capture reagent binds to VP3.
27. The kit of item 26, wherein the capture reagent binds to an epitope within residues 580 to 600 of VP3.
28. The kit of item 27, wherein the capture reagent binds to LQSANT (SEQ ID NO:1).
29. The kit of any one of items 18 to 28, wherein the detection reagent binds AAVx.
30. The kit of any one of items 18 to 28, wherein the detection reagent binds AAV8.
31. The kit of any one of items 18 to 30, wherein the detection reagent binds to a viral particle of the Anc80.
32. The kit of any one of items 18 to 31, further comprising a detectable moiety, and the detection reagent comprises a binding partner of the detectable moiety.
33. The kit of item 32, wherein the detection reagent comprises biotin, and the detectable moiety comprises horseradish peroxidase conjugated to avidin or streptavidin.
34. The kit of any one of items 18 to 33, wherein the capture reagent, the detection reagent, or both, are lyophilized.
35. The kit of any one of items 18 to 33, wherein the capture reagent, the detection reagent, or both, are provided in solution.
36. The kit of any one of items 18 to 35, further comprising one or more of a buffer, an assay stop solution, a calibration reagent, a detectable moiety capable of binding to the detection reagent, and a detectable substrate.
37. The kit of item 18, wherein the capture reagent binds AAV8, and the detection reagent binds AAVx.
38. A method of determining capsid titer of an adeno-associated virus in a cell suspension or cell lysate, the method comprising:
   (a) subjecting the cell suspension or cell lysate to high-performance liquid chromatography (HPLC), wherein the HPLC is performed with a resin that binds to a viral particle (VP) of the adeno-associated virus; and
   (b) detecting the VP in the cell suspension or cell lysate, thereby determining capsid titer of the adeno-associated virus.
39. The method of item 38, wherein the detecting comprises spectrophotometrically detecting capsid protein at 280 nm.
40. The method of item 38, wherein the adeno-associated virus comprises a fluorescent moiety, and the detecting comprises fluorescence detection.
41. The method of any one of items 38 to 40, wherein the cell lysate is an unpurified cell lysate or a partially purified cell lysate.
42. The method of any one of items 38 to 41, wherein the HPLC is performed with the resin contained in a column having a volume of about 0.1 to about 1.0 mL.
43. The method of any one of items 38 to 42, wherein the resin comprises an antibody or antigen-binding fragment thereof.
44. The method of any one of items 38 to 43, wherein the adeno-associated virus comprises Anc80.

## Claims

1. A method of determining capsid titer of an adeno-associated virus (AAV) in a cell suspension or cell lysate, the method comprising:
(a) subjecting the cell suspension or cell lysate to high-performance liquid chromatography (HPLC), wherein the HPLC is performed with a resin that binds to a viral particle (VP) of the adeno-associated virus; and
(b) detecting the VP in the cell suspension or cell lysate, thereby determining capsid titer of the adeno-associated virus.

2. The method of claim 1, wherein the detecting comprises spectrophotometrically detecting capsid protein at 270 nm to 290 nm, preferably 280 nm.

3. The method of claim 1 or claim 2, wherein the adeno-associated virus comprises a fluorescent moiety, and the detecting comprises fluorescence detection.

4. The method of claim 3, wherein the fluorescent moiety is a fluorescent dye, or wherein the fluorescent moiety is a fluorescent protein.

5. The method of any one of claims 1-4, wherein the cell lysate is an unpurified cell lysate or a partially purified cell lysate.

6. The method of any one of claims 1-5, wherein the cell lysate comprises one or more impurities selected from cellular components of the producer cell, a viral component that is not part of an assembled AAV particle, partially assembled AAV particles, a misfolded viral protein, and a malformed viral particle.

7. The method of any one of claims 1-6, wherein the cell suspension comprises AAV producer cells suspended in growth media or buffer.

8. The method of any one of claims 1-7, wherein the HPLC is performed with the resin contained in a column having a volume of about 0.1 mL to about 5.0 mL, preferably about 0.1 mL to about 1.0 mL.

9. The method of any one of claims 1-8, wherein the resin comprises an antibody or antigen-binding fragment thereof.

10. The method of any one of claim 1-9, wherein the resin binds a conformational epitope on a capsid of the AAV VP.

11. The method of any one of claims 1-10, wherein the AAV comprises Anc80.

12. The method of any one of claims 1-11, wherein the AAV capsid titer is determined by measuring peak area on a chromatogram of the HPLC.

13. The method of any one of claims 1-12, wherein the method further comprises measuring AAV genome titer by digital droplet PCR (ddPCR).

14. The method of claim 13, wherein the method further comprises determining a ratio of full to empty capsids in the cell suspension or cell lysate based on the AAV capsid titer measured by HPLC and the AAV genome titer measured by ddPCR.

15. The method of any one of claims 1-14, wherein the method is utilized in combination with a bioreactor.
